(19) Europäisches Patentamt
European Patent Office
Office européen des brevets

(11) Veröffentlichungsnummer : **0 167 770**
**B1**

(12) **EUROPÄISCHE PATENTSCHRIFT**

(45) Veröffentlichungstag der Patentschrift : **17.08.88**

(51) Int. Cl.⁴ : **C 07 D341/00**

(21) Anmeldenummer : **85106428.7**

(22) Anmeldetag : **24.05.85**

(54) **Verfahren zur Herstellung von 1,3,5-Trithian.**

(30) Priorität : **12.06.84 DE 3421702**

(43) Veröffentlichungstag der Anmeldung : **15.01.86 Patentblatt 86/03**

(45) Bekanntmachung des Hinweises auf die Patenterteilung : **17.08.88 Patentblatt 88/33**

(84) Benannte Vertragsstaaten : **BE DE FR GB NL**

(56) Entgegenhaltungen :
**AT-B- 269 889**
**CHEMICAL ABSTRACTS, Band 27, Nr. 1, 6. Juli 1970, Columbus, Ohio, USA. T. YAMAMURA "Tricyan(s-trithiane)" Seite 339, Spalte 2, Zusammenfassung-Nr. 3 944d**

(73) Patentinhaber : **CASSELLA Aktiengesellschaft**
**Hanauer Landstrasse 526**
**D-6000 Frankfurt am Main 61 (DE)**

(72) Erfinder : **Mix, Konrad, Dr.**
**Fuldaer Strasse 24**
**D-6000 Frankfurt am Main 61 (DE)**

(74) Vertreter : **Urbach, Hans-Georg, Dr. et al**
**Hanauer Landstrasse 526**
**D-6000 Frankfurt am Main 61 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 167 770 B1

**Beschreibung**

1,3,5-Trithian, auch kurz s-Trithian genannt, und seine Derivate können z. B. zur Herstellung von Insektiziden, Herbiziden, Fungiziden, Bakteriziden, Pestiziden sowie von Gerbstoffen, Flotationsmitteln, Antioxidantien und Korrosionsschutzmitteln herangezogen werden (vgl. E. Weißflog, Habilitationsschrift, Julius Maximilian Universität Würzburg (1981) Seite 10). Außerdem wird s-Trithian in Form des 2-Lithium-1,3,5-trithians als verkapptes nucleophiles Acylierungsmittel, z. B. bei der Herstellung von Aldehyden, benutzt (vgl. D. Seebach, Synthesis (1969), 17 bis 40 ; Org. Synthesis, Vol. 51 (1971) 39 bis 43).

Rohes s-Trithian kann dadurch hergestellt werden (vgl. Org. Synth. Vol. 16, 81), daß in ein Gemisch wäßriger Formalinlösung und konzentrierter, wäßriger Salzsäure (Molverhältnis 1 : 2,24) Schwefelwasserstoff eingeleitet wird. Der Ansatz wird durch ausgeschiedene Kristalle dick und muß von ihnen mehrfach durch Filtration befreit werden. Die Reaktionszeit wird mit 12 bis 24 Stunden veranschlagt. Die Rohausbeute liegt zwar bei 98 % der Theorie, das Produkt ist aber unrein und muß durch eine umständliche, absatzweise durchgeführte Extraktion mit Benzol gereinigt werden. Die Ausbeute sinkt dabei auf 92 bis 94 % der Theorie, während der Schmelzpunkt dabei lediglich von 210 bis 213 °C für das Rohprodukt auf 214 bis 215 °C ansteigt, obwohl der Literaturschmelzpunkt bei 219 bis 221 °C liegt. Dies ist darauf zurückzuführen, daß bei der absatzweise durchgeführten Extraktion mit Benzol zwar unlösliches Polymethylensulfid, nicht dagegen Verunreinigungen durch sauerstoffhaltige Ringsysteme entfernt werden, so daß das so hergestellte s-Trithian noch bis zu vier Gewichtsprozent Sauerstoff enthält (vgl. E. Weißflog loc. cit., Seiten 14 und 166).

Zur Herstellung eines « sauerstoffarmen » s-Trithians ist ein Verfahren bekannt (vgl. E. Weißflog loc. cit., Seite 167), bei dem in 50 %ige Schwefelsäure ein kräftiger Strom Schwefelwasserstoff eingeleitet wird und bei einer Temperatur von 70 °C langsam tropfenweise 30 %ige Formaldehydlösung im Verlauf von 3 Stunden zugefügt wird (Molverhältnis Formaldehyd : Schwefelsäure 1 : 2,86). Es wird ein rohes s-Trithian in einer Ausbeute von 96 % der Theorie erhalten, das immer noch knapp 2 % Sauerstoff enthält. Durch Auflösen des Rohprodukts in heißem Chloroform und Sättigen mit Petrolether kann in 78 % der Theorie ein Produkt mit einem Sauerstoffgehalt von 1 % erhalten werden.

Nach diesen bekannten Herstellungsverfahren für s-Trithian werden lediglich Produkte mit Sauerstoffgehalten von 1 bis 4 % erhalten, wobei das Produkt mit 1 % Sauerstoffgehalt nur nach einer umständlichen und verlustreichen Umfällung isoliert werden kann, wobei zudem ein schwierig zu trennendes Gemisch aus Chloroform und niedrig siedenden aliphatischen Kohlenwasserstoffen anfällt. Nachteilig ist ferner, daß bei dem mit Schwefelsäure arbeitenden Verfahren erhebliche Mengen Schwefelwasserstoff im Kreislauf durch das Reaktionsgemisch geführt werden müssen, um einen ständigen Überschuß an Schwefelwasserstoff aufrechtzuerhalten. Bei diesen bekannten Verfahren fallen zudem erhebliche Mengen anorganischer Abfallstoffe an, und zwar 1,75 kg HCl bei dem mit Salzsäure arbeitenden Verfahren und 8,15 kg Schwefelsäure bei dem mit Schwefelsäure arbeitenden Verfahren, jeweils bezogen auf 1 kg gereinigtes s-Trithian.

Zur Herstellung von s-Trithian ist es auch bekannt (vgl. Chemical Abstracts, Vol. 73, (1970), 3944 d), Schwefelwasserstoff in Wasser einzuleiten, das 1 % Schwefelsäure und 37 % Formaldehyd enthält, und die Mischung dann unter Druck auf 160 °C zu erhitzen. Das Arbeiten unter Druck ist technisch aufwendig. Außerdem wird s-Trithian lediglich in einer Ausbeute von 47 % und mit einem Schmelzpunkt von 214 bis 216 °C erhalten, was bedeutet, daß das Produkt noch verunreinigt ist.

Nach dem Verfahren der vorliegenden Erfindung wird in einem einstufigen einfachen Verfahren gut kristallisiertes, praktisch sauerstofffreies s-Trithian in praktisch quantitativer Ausbeute erhalten. Nach einer bevorzugten Ausführungsform können zudem die anfallenden Mengen anorganischer Abfallstoffe gegenüber den bisher bekannten Verfahren erheblich verringert werden.

Die Erfindung betrifft ein Verfahren zur Herstellung von 1,3,5-Trithian durch Umsetzung von Schwefelwasserstoff mit Formaldehyd in wäßriger Lösung in Gegenwart einer starken, nichtoxidierenden Säure bei Temperaturen über 30 °C. Das erfindungsgemäße Verfahren ist dadurch gekennzeichnet, daß die Umsetzung in einem Gemisch aus Wasser und einem inerten, in Wasser nicht oder nur wenig löslichen organischen Lösungsmittel durchgeführt wird.

Unter in Wasser nicht oder nur wenig löslichen organischen Lösungsmittel sind solche zu verstehen, die nach dem Vermischen mit Wasser ein Zweiphasensystem ausbilden. Derartige Lösungsmittel werden häufig auch als mit Wasser nicht mischbar bezeichnet. Die bei dem erfindungsgemäßen Verfahren verwendeten organischen Lösungsmittel müssen gegenüber den Reaktionskomponenten (Säure, Schwefelwasserstoff, Formaldehyd, s-Trithian) und Wasser inert sein und sollten keinen zu niedrigen und keinen zu hohen Siedepunkt besitzen. Zweckmäßigerweise sollten sie leicht mit Wasserdampf destillierbar sein. In der Regel liegt der Siedepunkt geeigneter organischer Lösungsmittel bei 50 bis 200 °C, vorzugsweise bei 80 bis 140 °C. Geeignete Lösungsmittel stammen z. B. aus folgenden Klassen : Aliphatische Kohlenwasserstoffe, wie z. B. n-Hexan, n-Heptan, n-Octan, n-Nonan, n-Decan, n-Undecan, 2-Methylhexan, 3-Methylhexan, 2,2-, 2,3-, 2,4-, 3,3-Dimethylpentan, 3-Ethylpentan, 2,2,3-Trimethylbutan, i-Octan, 2-Methylpentan, 3-Methylpentan, 2,2,4- und 2,3,4-Trimethylpentan, 2,2,5-Trimethylhexan, Spezialbenzine mit einem Siedepunkt von 60 bis 95 °C, 80 bis 110 °C, 100 bis 125 °C und 100 bis 140 °C, Testbenzin ; cycloaliphatische Kohlenwasserstoffe, wie z. B. Cyclopentan, Cyclohexan, Methylcyclopentan, Methylcyclohexan, 1,2-Dimethylcyclohexan, Dekalin, Tetralin ; aromatische Kohlenwasserstoffe, wie z. B. Benzol, Toluol, Ethylbenzol ; aliphatische und aromatische Halogenkohlenwasserstoffe, wie z. B. Chloroform, Tetrachlorkohlenwasserstoff, sec.-Butylchlorid, 1,2-Dichlorethan, 1,2-Dichlorpropan, 1,1,1-Trichlorethan, 1,1,2,2-Tetrachlorethan, Pentachlorethan, Chlorbenzol, o-Dichlorbenzol, Chlortoluol.

Vorzugsweise werden als organisches Lösungsmittel Chlorbenzol, Toluol oder von 80 bis 130 °C siedende Benzingemische verwendet. Auch Mischungen verschiedener inerter organischer Lösungsmittel können verwendet werden. Die Dichte des verwendeten Lösungsmittels oder Lösungsmittelgemischs kann gleich, größer oder kleiner als die der wäßrigen Phase des Reaktionsgemischs sein. Während der Durchführung der Reaktion werden die wäßrige und organische Phase durch Mischen gut ineinander verteilt, was z. B. durch Rühren erfolgen kann.

Der als Ausgangsprodukt benötigte Formaldehyd wird am einfachsten in Form der handelsüblichen 30 bis 40 %igen wäßrigen Lösungen eingesetzt. Die benötigten Formaldehydlösungen können jedoch auch auf beliebige andere Weise gewonnen werden, z. B. durch depolymerisierende Auflösung von Paraformaldehyd in Wasser mit Hilfe einer starken Säure. Hierfür wird zweckmäßigerweise eine Säure verwendet, wie sie ohnehin für die Durchführung des erfindungsgemäßen Verfahrens erforderlich ist, d. h. eine starke, nichtoxidierende Säure. Im Hinblick auf die leichte Oxidierbarkeit des Schwefelwasserstoffs muß die Säure nichtoxidierend sein. Es werden starke Säuren mit einem $p_{ka}$-Wert von gleich oder kleiner als 0 verwendet. Geeignete Säuren sind z. B. Chlorwasserstoffsäure, Bromwasserstoffsäure und Schwefelsäure oder Mischungen derartiger Säuren. Chlorwasserstoff- und Bromwasserstoffsäure werden zweckmäßigerweise in Form der handelsüblichen wäßrigen Lösungen eingesetzt, die bei der Salzsäure z. B. 30 bis 40 %ig sind. Auch Schwefelsäure wird normalerweise in Form von wäßrigen Lösungen eingesetzt, die z. B. 5 bis 15 normal sein können.

Ein Zusatz von Wasser ist nicht erforderlich, wenn die Säure und der Formaldehyd in den vorerwähnten handelsüblichen wäßrigen Lösungen eingesetzt werden. Bei der Herstellung von Formaldehyd durch Depolymerisation von Paraformaldehyd mit Salzsäure wird man nicht zu konzentriert arbeiten, da sich bei der Anwendung von warmer Salzsäure sonst unter Umständen der als cancerogen verdächtige Bis-(chlormethyl)-ether bilden kann. Umgekehrt ist eine zu große Verdünnung im Hinblick auf eine gute Raum-Zeit-Ausbeute unzweckmäßig.

Schwefelwasserstoff wird normalerweise gasförmig in das Reaktionsgemisch eingeleitet, kann jedoch auch im Reaktionsgemisch selbst, z. B. durch Zutropfen einer Sulfid- oder Hydrogensulfid-Lösung, z. B. einer Lösung eines Alkalimetall- oder Erdalkalimetallsulfhydrats, freigesetzt werden.

Bei der Durchführung des erfindungsgemäßen Verfahrens werden normalerweise der Formaldehyd und die Säure in Form von wäßrigen Lösungen und das in Wasser nicht oder nur wenig lösliche, inerte organische Lösungsmittel vorgelegt und, z. B. durch Rühren, durchmischt und in dieses Zweiphasensystem, dessen Phasen durch den Mischvorgang gut ineinander verteilt sind, Schwefelwasserstoff eingeleitet oder in ihm erzeugt. Durch die Reaktionswärme erwärmt sich der Reaktionsansatz rasch auf über 30 °C. Eine Kühlung ist normalerweise nicht erforderlich. Die Reaktion wird bei Temperaturen über 30 °C, vorzugsweise bei Temperaturen von 50 bis 100 °C, durchgeführt. Falls man die Reaktion in einem Autoklaven durchführt, kann sie auch bei Temperaturen bis ca. 150 °C durchgeführt werden. Durch die hohe Reaktionsgeschwindigkeit kann es beim Einleiten von Schwefelwasserstoff in das Reaktionsgemisch zu einem Zurücksteigen des Reaktionsgemischs in das Einleitungsrohr kommen. Dies kann durch geeignete Vorkehrungen, z. B. durch rasches Einleiten des Schwefelwasserstoffs oder durch Aufdrücken von Schwefelwasserstoff über dem Gasraum des Reaktionsgemischs vermieden werden.

Während der Reaktion kommt es zur zunehmenden Abscheidung von s-Trithian-Kristallen, wodurch das ursprünglich vorhandene 2-Phasensystem in ein 3-Phasensystem übergeht. Die Reaktion ist beendet, wenn keine weitere Schwefelwasserstoffaufnahme mehr erfolgt. Die Anwendung eines Schwefelwasserstoffüberschusses ist nicht schädlich.

Die Gesamtflüsssigkeitsmenge des Reaktionsgemischs soll ausreichen, um die gebildeten s-Trithian-Kristalle durch die angewandte Durchmischung während der Reaktion in gut beweglicher Suspension halten zu können. Normalerweise werden gute Ergebnisse erhalten, wenn 25 bis 400 Gew.% der erwarteten Ausbeute an s-Trithian an organischem Lösungsmittel eingesetzt werden. Wenn die Gesamtmenge des einzusetzenden Formaldehyds in Form einer wäßrigen Lösung vorgelegt wird, dann wird die Säuremenge so gewählt, daß auf 1 mol Formaldehyd mindestens 0,5 mol, vorzugsweise mindestens 0,8 mol Säure kommt. Die eingesetzte Säuremenge ist nach oben praktisch nicht begrenzt, so daß z. B. auf 1 mol Formaldehyd auch 3 oder 4 mol Säure eingesetzt werden können. Um den anorganischen Abfall zu vermindern, wird man jedoch in der Regel bestrebt sein, die Säuremenge gering zu halten. Das Molverhältnis Formaldehyd : Säure beträgt daher normalerweise 1 : (0,5 bis 2,2), insbesondere 1 : (0,8 bis 2,2), vorzugsweise 1 : (0,8 bis 1,2).

Um eine rasche Schwefelwasserstoffaufnahme und einen störungsfreien Verlauf der Reaktion sicherzustellen, ist es zweckmäßig, wenn die Konzentration der Säure in der wäßrigen Phase während der gesamten Reaktion mindestens 4 normal ist. Hierbei müssen selbstverständlich auch die Wassermengen für die Konzentrationsberechnung berücksichtigt werden, die in Form der wäßrigen Formaldehydlösung in das Reaktionsgemisch eingebracht werden und die während der Reaktion gemäß der Reaktionsgleichung

$$3CH_2O + 3H_2S \longrightarrow (CH_2S)_3 + 3H_2O$$

entstehen.

Der anorganische Abfall kann noch weiter erheblich verringert werden, wenn man die Säure in wäßriger Phase zusammen mit mindestens einem Teil des organischen Lösungsmittels vorlegt und in das durchmischte 2-Phasensystem Schwefelwasserstoff einleitet und unter Beibehaltung eines von Null verschiedenen Schwefelwasserstoffpartialdrucks Formaldehyd, z. B. in Form einer wäßrigen Lösung, oder als Paraformaldehyd, der z. B. in der restlichen Menge des organischen Lösungsmittels suspendiert ist, so einträgt, daß während der gesamten Reaktion das oben als untere Grenze angegebene Molverhältnis Formaldehyd : Säure von 1 : 0,5, vorzugsweise 1 : 0,8, nicht unterschritten wird.

Die Isolierung des gebildeten s-Trithians kann auf verschiedene Weise erfolgen. Das Produkt kann z. B. durch Absaugen, Druckfiltration, Abschleudern oder dgl. abgetrennt, mit Wasser nachgewaschen und zur Entfernung von eventuell anhaftenden Lösungsmittelresten ausgedämpft und anschließend getrocknet werden. Diese Isolierung liefert, insbesondere wenn sie in der Wärme durchgeführt wird, das reinste s-Trithian des erfindungsgemäßen Verfahrens mit Ausbeuten von ca. 94 % der Theorie. Das anfallende flüssige 2-Phasengemisch wird im Flüssig-flüssig-Abscheider oder mittels Wasserdampfdestillation getrennt.

Zur Isolierung des s-Trithians kann auch der Ansatz zunächst durch Wasserdampfdestillation von dem organischen Lösungsmittel befreit werden, ehe das s-Trithian z. B. durch Filtration abgetrennt wird. Bei dieser Art der Isolierung werden Ausbeuten von über 96 % der Theorie erreicht.

Das zurückgewonnene organische Lösungsmittel kann wiederverwendet werden. Die bei dem erfindungsgemäßen Verfahren erhaltenen s-Trithian-Produkte sind kristallin und besitzen Schmelzpunkte zwischen 218 und 221 °C und entsprechen somit den Literaturangaben. Der Gehalt an nicht sublimierbaren Polymeren liegt, wenn die Reaktion im bevorzugten Temperaturbereich über 50 °C und bei dem bevorzugten Molverhältnis Formaldehyd : Säure von über 1 : 0,8 durchgeführt wird, im allgemeinen unter 0,5 % und höchstens bei 1 %, steigt allerdings etwas an, wenn die Reaktion zwischen 30 und 50 °C oder mit Molverhältnissen Formaldehyd : Säure von 1 : (0,5 bis 0,8) durchgeführt wird. Die Elementaranalysen ergeben für die Summe aus Kohlenstoff, Wasserstoff und Schwefel Werte, die praktisch bei 100 % liegen, so daß die Produkte praktisch frei von Sauerstoff enthaltenden Produkten sind. Auch unter Berücksichtigung der Analysenungenauigkeit können die erfindungsgemäß hergestellten Produkte höchstens 0,5 % Sauerstoff enthalten. Daß eine derartige Reinheit bei derart hoher Ausbeute in einem einstufigen Verfahren ohne Reinigungsoperation erhalten werden kann, muß als überraschend bezeichnet werden. Ferner war es überraschend, daß der anorganische Abfall gegenüber den bisher bekannten Verfahren vermindert, nach der besonderen Ausgestaltung des erfindungsgemäßen Verfahrens, bei welcher Formaldehyd oder Paraformaldehyd dem Reaktionsgemisch zudosiert wird, sogar auf minimale Werte, wie z. B. 87 g HCl pro kg s-Trithian und gegebenenfalls noch weniger, vermindert werden kann.

## Beispiel 1

In einem 4-Liter-Glaskolben mit Rührer, Thermometer, Gaseinleitungs- und -ableitungsrohr, letzteres mit Rückschlagventil und Mündung in einer Sperrflüssigkeit, werden 330 g 40 %iger Formaldehyd (4,40 mol), 770 ml 36 %ige Salzsäure (9,0 mol) und 1 l Chlorbenzol bei Raumtemperatur vorgelegt. Unter starkem Rühren wird Schwefelwasserstoff in starkem Strome eingeleitet, wobei nur zu Beginn des Einleitens Gas durch die Sperrflüssigkeit hindurch entweichen sollte. Durch die Reaktionswärme heizt sich das Gemisch rasch auf über 50 °C auf. Mittels eines Wasserbads wird die Temperatur im Reaktionsgemisch zwischen 50 und 60 °C gehalten. Nach ein bis drei Stunden — je nach Stärke des Schwefelwasserstoffstroms — ist der Ansatz mit Schwefelwasserstoff gesättigt, was sich durch Gasdurchgang durch die Sperrflüssigkeit anzeigt. Es wird noch kurze Zeit unter weiterem schwachen Durchleiten von Schwefelwasserstoff bei 50 bis 60 °C gerührt, dann wird das ausgeschiedene Festprodukt noch warm über eine Gassinternutsche abfiltriert, mit warmen Wasser neutral gewaschen und bei erhöhter Temperatur getrocknet. Man erhält 190,3 g (93,9 % der Theorie) s-Trithian als weißes kristallines Pulver vom Schmelzpunkt 218 bis 221 °C. Der sublimierbare Anteil beträgt 99,9 % und der anorganische Abfall 1,72 kg HCl je 1 kg s-Trithian.

| | | | |
|---|---|---|---|
| Analyse berechnet : $(CH_2S)_3$ | C 26,1 % | H 4,4 % | S 69,6 % |
| gefunden : | C 26,5 % | H 4,2 % | S 69,4 % |

## Beispiel 2

Bei einer Wiederholung des Beispiels 1 wird der Ansatz wie folgt aufgearbeitet :

Aus dem erhaltenen 3-Phasengemisch wird das Chlorbenzol durch Wasserdampf abgetrieben und praktisch quantitativ wiedergewonnen. Die wäßrige Trithiansuspension wird heiß abfiltriert ; das grobkristalline Produkt wird mit Wasser neutral gewaschen und anschließend getrocknet. Man erhält 193,5 g s-Trithian. Nach Abkühlen der Mutterlauge können aus dieser nochmals 1,1 g erhalten werden.

Insgesamt entspricht das einer Ausbeute von 96 % der Theorie. Der Schmelzpunkt liegt bei 217 bis 219 °C, der sublimierbare Anteil bei 98,7 %. Der anorganische Abfall beträgt hier 1,68 kg HCl je 1 kg s-Trithian.

Beispiel 3

330 g 40 %iges Formaldehyd (4,4 mol), 330 ml 63 %ige Bromwasserstoffsäure (4,4 mol) und 600 ml Chlorbenzol werden in der in Beispiel 1 beschriebenen Apparatur unter Rühren bei 50 bis 75 °C mit Schwefelwasserstoff gesättigt, anschließend wird das Chlorbenzol mit Wasserdampf abgetrieben und das ausgefallene s-Trithian nach dem Erkalten abfiltriert, neutral gewaschen und getrocknet. Man erhält 200,4 g (98,9 % der Theorie) in Form weißer Kristalle vom Schmelzpunkt 219 bis 221 °C mit einem nichtsublimierbaren Anteil von 0,8 %. Der anorganische Abfall beträgt 1,78 kg HBr je 1 kg s-Trithian.

Beispiel 4

354 g 36,3 %ige Salzsäure (3,52 mol), 334 g 39,55 %iger Formaldehyd (4,4 mol) und 600 ml Chlorbenzol werden in der in Beispiel 1 beschriebenen Apparatur unter Rühren bei 50 bis 54 °C mit Schwefelwasserstoff in 3 bis 4 Stunden gesättigt. Dann wird das Chlorbenzol mit Wasserdampf abgetrieben und das Produkt nach Erkalten abgesaugt, mit Wasser gewaschen und getrocknet. Man erhält 199 g (98,2 % der Theorie) s-Trithian als weißes, kristallines Pulver mit einem nichtsublimierenden Anteil von 0,2 % und einem Schmelzpunkt von 220 °C. Gemäß der CHS-Bestimmung ist die Substanz frei von Sauerstoff. Der anorganische Abfall beträgt 0,65 kg HCl je 1 kg s-Trithian.

Beispiel 5

Bei einer Wiederholung des Beispiels 4 werden anstelle von 354 g 36,3%ige Salzsäure 442 g 36,3 %ige Salzsäure (4,4 mol) und anstelle von 600 ml Chlorbenzol nur 60 ml Chlorbenzol eingesetzt. Man erhält 198,3 g ( = 97,8 % der Theorie) s-Trithian als weiße Kristalle vom Schmelzpunkt 218 bis 219 °C mit einem sublimierbaren Anteil von 98,7 %. Das Produkt ist gemäß der CHS-Bestimmung frei von Sauerstoff. Der anorganische Abfall beträgt 0,81 kg HCl je 1 kg s-Trithian.

Beispiel 6

Man verfährt wie in Beispiel 5, nimmt aber statt des Chlorbenzols ein bei 80 bis 110 °C siedendes Alkangemisch. Man erhält 199,6 g ( = 98,6 % der Theorie) s-Trithian als weiße Kristalle vom Schmelzpunkt 220 °C.

Analyse : C 26,2 % H 4,2 % S 69,5 %.

Der anorganische Abfall beträgt 0,8 kg HCl je 1 kg s-Trithian.

Beispiel 7

Man verfährt wie in Beispiel 5, nimmt aber statt 60 ml Chlorbenzol 600 ml Toluol. Man erhält 197,6 g, entsprechend 97,5 % der Theorie, an weißem, kristallinem s-Trithian vom Schmelzpunkt 218 °C mit einem sublimierbaren Anteil von 99,0 % und der folgenden Analyse :

C 26,6 % H 4,3 % S 70,1 %.

Der anorganische Abfall beträgt 0,81 kg HCl je 1 kg s-Trithian.

Beispiel 8

Man verfährt wie in Beispiel 5, nimmt aber statt 60 ml Chlorbenzol 600 ml und führt die Reaktion bei 89 bis 91 °C aus. Man erhält 188,6 g ( = 93,0 % der Theorie) weißes kristallines s-Trithian vom Schmelzpunkt 220 °C mit einem nichtsublimierbaren Anteil von unter 0,1 %. Der anorganische Abfall beträgt 0,85 kg HCl je 1 kg s-Trithian.

Beispiel 9

Man verfährt wie in Beispiel 8, führt die Reaktion jedoch in einem geschlossenen Gefäß bei 110 °C durch. Man erhält 200,7 g ( = 98,9 % der Theorie) weißes kristallines s-Trithian vom Schmelzpunkt 220 °C. Der anorganische Abfall beträgt 0,79 kg HCl je 1 kg s-Trithian.

Beispiel 10

Man verfährt wie in Beispiel 5, nimmt aber nur 217 g 37,0 %ige Salzsäure (2,2 mol) statt 442 g 36,3 %iger Salzsäure. Man erhält 183,2 g ( = 90,4 % der Theorie) weißes kristallines s-Trithian vom Schmelzpunkt 220 °C mit einem nichtsublimierenden Anteil von 5,1 %. Der anorganische Abfall beträgt 0,44 kg je 1 kg s-Trithian.

Beispiel 11

Man verfährt wie in Beispiel 5, führt die Reaktion aber bei 33 bis 34 °C durch. Man erhält 199,8 g ( = 98,6 % der Theorie) an einem weißen kristallinen Pulver vom Schmelzpunkt 219 bis 220 °C mit einem nichtsublimierenden Anteil von 7,5 %. Der anorganische Abfall beträgt 0,80 kg HCl je 1 kg s-Trithian.

Beispiel 12

Man verfährt wie in Beispiel 1, nimmt aber 600 ml Chlorbenzol und leitet keinen Schwefelwasserstoff in die salzsaure Formaldehydlösung ein, sondern tropft im Verlauf von 2 Stunden 840 g 30 %ige Natriumsulfhydratlösung zu, wobei darauf geachtet wird, daß außer zu Beginn und Ende der Reaktion kein Schwefelwasserstoff durch die Sperrflüssigkeit durchschlägt und entweicht. Zur Aufarbeitung wird das Chlorbenzol mit Wasserdampf abgetrieben und das s-Trithian nach Erkalten abgesaugt, gründlich mit Wasser gewaschen und getrocknet. Man erhält 202 g weiße Kristalle ( = 99,7 % der Theorie) an s-Trithian vom Schmelzpunkt 216 bis 218 °C mit einem sublimierenden Anteil von 98 %.

Der anorganische Abfall beträgt 1,25 kg NaCl und 0,81 kg HCl je 1 kg s-Trithian.

Beispiel 13

In einem 1-Liter-Glaskolben mit Rührer, Thermometer, Gaseinleitungsrohr, Rückflußkühler und Gasableitungsrohr mit Rückschlagventil und Mündung in einer Sperrflüssigkeit werden 97,2 g 36,3 %ige Salzsäure (0,92 mol), 30 g Paraformaldehyd und 60 ml Chlorbenzol bei 75 bis 80 °C vorgelegt, wobei der Paraformaldehyd depolymerisiert. Dann wird unter starkem Rühren in raschem Strom Schwefelwasserstoff eingeleitet. Sobald sich die Schwefelwasserstoffaufnahme verlangsamt, was am Flüssigkeitsspiegel in der Sperrflüssigkeit abzulesen ist, wird damit begonnen, eine Suspension von 102 g Paraformaldehyd in 300 ml Chlorbenzol so zuzudrücken, daß in der Apparatur nur ein geringer Unterdruck bis ein geringer Überdruck bei konstantem Schwefelwasserstoffstrom herrscht. Sobald die Paraformaldehydsuspension zugegeben und die Sättigung mit Schwefelwasserstoff beendet ist, wird noch bei schwachem Schwefelwasserstoffüberdruck 15 bis 20 Minuten bei 75 bis 80 °C nachgerührt. Anschließend wird der Ansatz wie in Beispiel 3 beschrieben aufgearbeitet. Man erhält 195 g ( = 96,5 % der Theorie) weißes kristallines s-Trithian vom Schmelzpunkt 218 bis 220 °C mit einem sublimierbaren Anteil von ca. 99 %. Der anorganische Abfall beträgt 0,18 kg HCl je 1 kg s-Trithian.

Zu einem entsprechenden Ergebnis gelangt man, wenn man, was beim Arbeiten in größerem Maßstab empfehlenswert ist, die Reaktion im geschlossenen Druckgefäß durchführt und ihren Fortgang am Gasdruck abliest.

Beispiel 14

Man verfährt wie in Beispiel 13, verwendet aber nur eine Menge von 48 g 36,3 %iger Salzsäure, legt 10 g Paraformaldehyd vor und dosiert später 122 g anstelle von 102 g Paraformaldehyd zu. Die Schwefelwasserstoffaufnahme erfolgt bei sonst gleichen Bedingungen langsamer als bei Beispiel 13. Es werden 199,8 g ( = 98,7 % der Theorie) an gut kristallisiertem s-Trithian erhalten. Dessen sublimierbarer Anteil beträgt 99 %, die Menge des anorganischen Abfalls liegt bei 87 g HCl je 1 kg s-Trithian.

Beispiel 15

Ein Gemisch aus 76 g 39,5 %igem wäßrigen Formaldehyd (1 mol), 200 ml 10 n Schwefelsäure (2 mol) und 100 ml Chlorbenzol wird bei 75 °C unter starkem Rühren in ca. 22 Minuten mit Schwefelwasserstoff bei einem Gasfluß von ca. 60 bis 65 l/h gesättigt. Dann wird das Chlorbenzol mit Wasserdampf abgetrieben und nach dem Erkalten das ausgefallene s-Trithian auf einer Filternutsche gesammelt, neutral gewaschen und getrocknet. Man erhält 45,3 g ( = 98,5 % der Theorie) s-Trithian in Form weißer Kristalle vom Schmelzpunkt 219 bis 221 °C und mit einem sublimierbaren Anteil von 99,4 %.

Beispiel 16

In einen evakuierten 0,7 l Tantalautoklaven werden unter Rühren nacheinander 120 ml 36 %ige Salzsäure, 120 ml 40 %ige wäßrige Formaldehydlösung und 240 ml Chlorbenzol eingesaugt. Die Temperatur wird auf 50 °C gebracht und Schwefelwasserstoffgas unter starkem Rühren bei leicht erhöhtem Druck — es genügen 0,5 bis 2 bar — aufgedrückt. Nach ca. 15 min ist die Schwefelwasserstoffaufnahme praktisch beendet, die Temperatur ist auf ca. 80 °C angestiegen. Man läßt noch ca. 30 min. bei

leicht erhöhtem Schwefelwasserstoffdruck nachrühren, um sicherzustellen, daß sämtlicher Formaldehyd umgesetzt ist. Dann wird wie in Beispiel 2 beschrieben aufgearbeitet. Man erhält 78,1 g entsprechend 96,3 % der Theorie an s-Trithian vom Fp 222 °C und mit einem sublimierbaren Anteil von 99,2 %.

## Patentansprüche

1. Verfahren zur Herstellung von 1,3,5-Trithian durch Umsetzung von Schwefelwasserstoff mit Formaldehyd in wäßriger Lösung in Gegenwart einer starken, nichtoxidierenden Säure bei Temperaturen über 30 °C, dadurch gekennzeichnet, daß die Umsetzung in einem Gemisch aus Wasser und einem inerten, in Wasser nicht oder nur wenig löslichen organischen Lösungsmittel durchgeführt wird.

2. Verfahren nach Anspruch 1, dadurch gekennzeichnet, daß die Reaktion bei Temperaturen von 30 °C bis 150 °C durchgeführt wird.

3. Verfahren nach Anspruch 1 oder 2, dadurch gekennzeichnet, daß die Reaktion bei Temperaturen von 50 °C bis 100 °C durchgeführt wird.

4. Verfahren nach einem oder mehreren der Ansprüche 1 bis 3, dadurch gekennzeichnet, daß ein organisches Lösungsmittel mit einem Siedepunkt von 50 bis 200 °C verwendet wird.

5. Verfahren nach einem oder mehreren der Ansprüche 1 bis 4, dadurch gekennzeichnet, daß ein organisches Lösungsmittel mit einem Siedepunkt von 80 bis 140 °C verwendet wird.

6. Verfahren nach einem oder mehreren der Ansprüche 1 bis 5, dadurch gekennzeichnet, daß als starke nichtoxidierende Säure eine solche mit einem $P_{ka}$-Wert von gleich oder kleiner 0 verwendet wird.

7. Verfahren nach einem oder mehreren der Ansprüche 1 bis 6, dadurch gekennzeichnet, daß während der gesamten Reaktion ein Molverhältnis Formaldehyd : Säure von mindestens 1 : 0,5, vorzugsweise von mindestens 1 : 0,8 eingehalten wird.

8. Verfahren nach einem oder mehreren der Ansprüche 1 bis 7, dadurch gekennzeichnet, daß während der gesamten Reaktion ein Molverhältnis Formaldehyd : Säure = 1 : (0,8 bis 2,2), vorzugsweise 1 : (0,8 bis 1,2) eingehalten wird.

9. Verfahren nach einem oder mehreren der Ansprüche 1 bis 8, dadurch gekennzeichnet, daß während der gesamten Reaktion in der wäßrigen Phase eine mindestens 4 normale Säurekonzentration eingehalten wird.

10. Verfahren nach einem oder mehreren der Ansprüche 1 bis 9, dadurch gekennzeichnet, daß im Verlauf der Reaktion Formaldehyd in das Reaktionsgemisch eingebracht oder in ihm erzeugt wird.

## Claims

1. Process of preparing 1,3,5-trithiane by reacting hydrogen sulphide with formaldehyde in aqueous solution in the presence of a strong non-oxidizing acid at temperatures of over 30 °C, characterized in that the reaction is carried out in a mixture of water and an inert organic solvent that is insoluble in water or only slightly soluble in water.

2. The process of claim 1, characterized in that the reaction is carried out at temperatures of between 30 and 150 °C.

3. The process of claim 1 or 2, characterized in that the reaction is carried out at temperatures of between 50 and 100 °C.

4. The process of one or several of claims 1 to 3, characterized in that the organic solvent used has a boiling point of 50 to 200 °C.

5. The process of one or several of claims 1 to 4, characterized in that the organic solvent used has a boiling point of 80 to 140 °C.

6. The process of one or several of claims 1 to 5, characterized in that the strong non-oxidizing acid used has a $P_{Ka}$ of equal to or smaller than 0.

7. The process of one or several of claims 1 to 6, characterized in that a molar ratio of formaldehyde : acid of at least 1 : 0.5, preferably of at least 1 : 0.8, is maintained during the entire reaction.

8. The process of one or several of claims 1 to 7, characterized in that a molar ratio of formaldehyde : acid of 1 : (0.8 to 2.2), preferably 1 : (0.8 to 1.2), is maintained during the entire reaction.

9. The process of one or several of claims 1 to 8, characterized in that in the aqueous phase an at least four times the normal acid concentration is maintained during the entire reaction.

10. The process of one or several of claims 1 to 9, characterized in that, in the course of the reaction, formaldehyde is introduced in the reaction mixture or is produced therein.

## Revendications

1. Procédé pour préparer le trithiane-1,3,5 par réaction, à des températures supérieures à 30 °C, de l'hydrogène sulfuré avec le formaldéhyde en solution aqueuse en présence d'un acide fort non oxydant, procédé caractérisé en ce qu'on conduit la réaction dans un mélange d'eau et d'un solvant organique

inerte, insoluble ou peu soluble seulement dans l'eau.

2. Procédé selon la revendication 1, caractérisé en ce qu'on conduit la réaction à des températures de 30 °C à 150 °C.

3. Procédé selon la revendication 1 ou 2, caractérisé en ce qu'on conduit la réaction à des températures de 50 °C à 100 °C.

4. Procédé selon une ou plusieurs des revendications 1 à 3, caractérisé en ce qu'on utilise un solvant organique ayant un point d'ébullition de 50 à 200 °C.

5. Procédé selon une ou plusieurs des revendications 1 à 4, caractérisé en ce qu'on utilise un solvant organique ayant un point d'ébullition de 80 à 140 °C.

6. Procédé selon une ou plusieurs des revendications 1 à 5, caractérisé en ce qu'on utilise comme acide fort non oxydant un tel acide ayant un $p_{ka}$ égal ou inférieur à 0.

7. Procédé selon une ou plusieurs des revendications 1 à 6, caractérisé en ce que l'on maintient pendant la totalité de la réaction un rapport molaire formaldéhyde : acide au moins égal à 1 : 0,5 et de préférence au moins égal à 1 : 0,8.

8. Procédé selon, une ou plusieurs des revendications 1 à 7, caractérisé en ce qu'on maintient pendant la totalité de la réaction un rapport molaire formaldéhyde : acide égal à 1 : (0,8 à 2,2), et de préférence égal à 1 : (0,8 à 1,2).

9. Procédé selon une ou plusieurs des revendications 1 à 8, caractérisé en ce qu'on maintient pendant la totalité de la réaction une concentration d'acide en phase aqueuse d'au moins 4 fois la concentration normale.

10. Procédé selon une ou plusieurs des revendications 1 à 9, caractérisé en ce que, au cours de la réaction, on introduit le formaldéhyde dans le mélange réactionnel ou on le produit dans celui-ci.